# EUROPÄISCHE PATENTSCHRIFT

(11) **EP 2 193 376 B1**
(45) Veröffentlichungstag und Bekanntmachung des Hinweises auf die Patenterteilung: **22.08.2012**
(21) Anmeldenummer: 08802358.5
(22) Anmeldetag: 11.09.2008
(51) Int. Cl.: G01N 33/68

(54) **LEKTINBASIERTER GLYKAN-ASSAY**
LECTIN-BASED GLYCAN ASSAY
DOSAGE DE POLYSACCHARIDE PAR LECTINE

(30) Priorität: 11.09.2007 EP 07075800
(43) Veröffentlichungstag der Anmeldung: 09.06.2010
(73) Patentinhaber: GALAB Technologies GmbH, 21502 Geesthacht (DE)
(72) Erfinder: KUBALLA, Jürgen, 21502 Geesthacht (DE); UMNUS, Kirstin, 21502 Geesthacht (DE); CARTELLIERI, Simone, 21502 Geesthacht (DE)
(74) Vertreter: Boeckh, Tobias
(86) Internationale Anmeldenummer: PCT/EP2008/007838
(87) Internationale Veröffentlichungsnummer: WO 2009/033753

(56) Entgegenhaltungen:
- EP-A- 1 460 425
- EP-A2- 0 662 611
- WO-A-03/087821
- WO-A-2004/040307
- WO-A1-89/04490
- LUNDY F T ET AL: "AN ANTIBODY-LECTIN SANDWICH ASSAY FOR QUANTIFYING PROTEIN GLYCOFORMS" MOLECULAR BIOTECHNOLOGY, TOTOWA, NJ, US, Bd. 12, Nr. 2, September 1999 (1999-09), Seiten 203-206, XP009033133 ISSN: 1073-6085
- ANONYMOUS: "Alkaline Phosphatase (E. coli C75) from GE Healthcare, formerly Amersham Biosciences" BIO-MEDICINE PRODUCTS INFO, [Online] XP002502744 Gefunden im Internet: URL:http://www.bio-medicine.org/biology-pr oducts/Alkaline-Phosphatase--28E--coli-C75 -29-from-GE-Healthcare--formerly-Amersham- Biosciences-3754-1/> [gefunden am 2008-11-06]

## Beschreibung

Die Erfindung betrifft die Verwendung von deglykosylierten Detektoren, insbesondere sekundären Antikörpern, zur Bestimmung der Zuckerstrukturen von Proteinen, insbesondere rekombinanten Proteinen. Sie betrifft weiterhin insbesondere die Verwendung von deglykosylierten Enzymen bei der Bestimmung von Zuckerstrukturen mithilfe einer Enzym-Substrat-Reaktion; des weiteren betrifft die Erfindung ein Verfahren zur Bestimmung der Zuckerstrukturen von Proteinen sowie einen Zuckerbestimmungskit und die Verwendung des Kits zur Bestimmung von Zuckerstrukturen, insbesondere von rekombinanten therapeutischen Proteinen, bevorzugt Immunoglobulinen.

Rekombinante therapeutische Proteine sind im Stand der Technik bekannt. Im Stand der Technik werden beispielsweise humane Proteine, wie das Erythropoeitin oder Antikörper, rekombinant in CHO-Zellen hergestellt. Diese Technik stellt zwar sicher, dass die in der DNA kodierte Aminosäuresequenz des Proteins reproduziert werden kann. Die posttranslationalen Veränderungen des rekombinanten Proteins, wie die Glykosylierung, sind allerdings in Abhängigkeit vom Zellclon und den Kulturbedingungen variabel. Bei der Bereitstellung von rekombinanten Proteinen, insbesondere therapeutischen Proteinen, ist zu berücksichtigen, dass die Zuckerstrukturen bzw. die Protein-Glykosylierung dazu führt, dass das Protein sich gewebe-, zell-, entwicklungs-, und/oder speziesspezifisch und -typisch in Abhängigkeit von der Glykosylierung verhält. Wesentliche strukturelle Eigenschaften, die eine zentrale Bedeutung beim Einsatz in der Therapie haben, werden durch die Zuckerstrukturen der Proteine determiniert. Bekannte rekombinante therapeutische Proteine sind beispielsweise vollständige, d. h. intakte, monoklonale Antikörper.

Im Stand der Technik ist noch kein Verfahren beschrieben, welches es schnell und automatisierbar ermöglicht, Zuckerstrukturen, insbesondere an rekombinanten therapeutischen Proteinen, wie z. B. intakten monoklonalen Antikörpern, zu bestimmen.

Bisher werden im Stand der Technik aufwendige Verfahren wie HPLC, MS oder CE nach Abspaltung der Zucker verwendet. Dazu ist es notwendig, die Zuckerstrukturen mittels chemischer oder enzymatischer Hydrolyse vom Protein zu entfernen. Die enzymatischen Verfahren lassen zwar eine selektive Abspaltung bestimmter Strukturen zu, sind aber in der Durchführung aufwendig und anfällig für Störungen. Im Anschluss an die Abspaltung der Zucker muss eine Abtrennung vom Protein erfolgen, die häufig säulenchromatographisch erfolgt. Diese Vorbereitungschritte und die oftmals geringe Empfindlichkeit der anschließenden Analyseverfahren führen dazu, dass eine nicht geringe Menge des rekombinanten Proteins (500-1000 µg) benötigt wird, was in der Praxis einen Nachteil darstellt. Die Bestimmung der Zuckerstrukturen erfordert nicht selten die Kombination mehrerer analytischer Verfahren, wie zum Beispiel eine massenspektrometrische Untersuchung mittels MALDI-MS, welche qualitative Ergebnisse liefert und zwischen Hexosen nicht differenzieren kann, und eine quantitative Bestimmung definierter Zucker mittels HPLC oder photometrischer Verfahren. Der Einsatz der letztgenannten Verfahren ist vom Vorhandensein reiner Glykane als Vergleichsstandards abhängig.

Der Stand der Technik offenbart unter anderem eine Methode zur Detektion des terminalen Glykosylierungsmusters von IgG. Hierzu wird eine Probe, die das Protein, welches untersucht werden soll, umfasst, auf Nitrocellulose immobilisiert. Anschließend werden die Proteine, bevorzugt IgG, so behandelt, dass die Zuckerreste (Gal, GlcNAc) freigesetzt werden. Die Detektion der IgGs in der Probe erfolgt mit markierten Lektinen, wobei die Markierung wiederum mit Enzymen erfolgt (WO 89/04490).

Eine weitere Methode des Standes der Technik umfasst die Immobilisierung von Lektinen auf einer Platte und die Detektion mit sekundärem Antikörper-Enzym-Konjugat. Das Lektin ist hierbei so ausgewählt, dass es nur eine abnorme Zuckerstruktur erkennen kann; es ist daher nicht geeignet, IgGs mit Galactose, Fukose und anderen Zuckern zu bestimmen (WO 98/16825).

Weiterhin ist im Stand der Technik eine Methode zur Detektion eines Glykoproteins mit markierten Lektinen beschrieben, bei der zunächst ein sogenannter Fänger-Antikörper (Fab-Teil) immobilisiert wird. Dessen Fc-Teil wird abgespalten, um Zucker, die den Messvorgang behindern können, zu verwerfen. Nachteilhafterweise wird in diesem Verfahren (WO 03/087821) das Problem der Fab-Glykosylierung nicht gelöst und weiterhin die Glykosylierung des Enzyms bei der Bestimmung der Zuckerstrukturen nicht berücksichtigt.

In der EP 0 399 464 A2 werden fünf Methoden offenbart, bei denen spezifische Zuckerreste mithilfe von Kunststoffpartikeln bestimmt werden. Diese Methode erlaubt wegen möglicher Zuckerstrukturen an den eingesetzten Substanzen keine zufriedenstellenden Ergebnisse, ebenso wie das Verfahren gemäß EP 0 662 611 A2, bei welchem Glykokonjugate im Sandwichaufbau mit Lektinen untersucht werden, indem zunächst ein Fänger-Antikörper immobilisiert wird, der chemisch behandelt vorliegt, um den Zucker zu inaktivieren und falsche Hintergrundsignale zu vermeiden.

Weiterer Stand der Technik ergibt sich aus Lundy et al., 1999, WO 2004/040307, EP 1 460 452 und der WO 03/087831, die verschiedene Assays zur Detektion von Glykoproteinen beschreiben. Nachteilhafterweise erlauben diese keine gute Bestimmung von Zuckerstrukturen von Proteinen, da das einzelne Messsignal sich nicht ausreichend von dem Hintergrundrauschen unterscheidet.

Alle Methoden des Standes der Technik sind nicht geeignet, Zuckerstrukturen vor allem bei rekombinanten Proteinen wie Immoglobulinen, insbesondere IgG sicher bzw. effektiv zu bestimmen.

Im Lichte des Standes der Technik war es völlig überraschend, dass die Verwendung eines deglykosylierten Detektionssystems zur Bestimmung mindestens einer Zuckerstruktur, bevorzugt von allen Zuckerstrukturen von Proteinen, insbesondere von rekombinanten, bevorzugt von rekombinanten, immobilisierten Proteinen verwendet werden kann, wobei das Detektionssystem ein deglykosyliertes Markerenzym und eine Bindestruktur umfasst und die Bindestrukturen bevorzugt mindestens sekundäre Antikörper, Protein A und/oder Fragmente hiervon beinhalten oder umfassen und die bevorzugt rekombinanten Proteine Erkennungsmoleküle, bevorzugt primäre Antikörper sind, ausgewählt aus der Gruppe umfassend IgM, IgE, IgD, IgA, IgY und/oder IgW, bevorzugt IgG. Es war völlig überraschend, dass die erfindungsgemäße Lehre verwendet werden kann, um Zuckerstrukturen von rekombinanten Proteinen wie Immunglobulinen (primären Antikörpern) sicher und effektiv zu bestimmen. Insbesondere war es überraschend, dass sehr gute Resultate bei der Bestimmung von Zuckerstrukturen erzielt werden können, wenn neben dem Markerenzym auch die Bindestrukturen wie der sekundäre Antikörper bzw. Nachweisantikörper deglykosyliert vorliegen.

Das erfindungsgemäße Detektionssystem bzw. umfasst zum einen ein deglykosyliertes Enzym bzw. Markerenzym und deglykosylierte Bindestrukturen, das heißt Detektoren, die beispielsweise Protein A, sekundäre Antikörper und/oder Fragmente hiervon sein können. Da erfindungsgemäß bevorzugt primäre Antikörper untersucht werden sollen, ist dem durchschnittlichen Fachmann bekannt, welche Bindestrukturen er einsetzen kann, um die bevorzugt primären Antikörper zu untersuchen. Dem Fachmann ist bekannt, dass er hierzu insbesondere sekundäre Antikörper, aber beispielsweise auch Protein A beziehungsweise Protein G oder Fragmente sekundärer Antikörper oder Bruchstücke von Protein A oder Protein G verwenden kann. Die genannten Untersuchungsmoleküle (Markerenzym und Bindestrukturen) müssen deglykosyliert vorliegen.

Die Erfindung betrifft demgemäß die überraschende Lehre, bei der Bestimmung von Zuckerstrukturen von insbesondere rekombinanten Proteinen deglykosylierte Detektoren und Enzyme zur Markierung (Markerenzyme) bzw. ein deglykosyliertes Detektionssystem zur Bestimmung von Zuckerstrukturen einzusetzen, wobei das Detektionssystem bevorzugt sekundäre Antikörper, Protein A, Protein G und/oder Fragmente hiervon als Detektor neben den deglykosylierten Markerenzymen umfasst und die rekombinanten Proteine Erkennungsmoleküle sind, bevorzugt ausgewählt aus der Gruppe der IgG-, IgM-, IgE-, IgD-, IgA-, IgY- und/oder IgB-Antikörper, insbesondere IgG. Insbesondere bei der Bestimmung der Zuckerstrukturen von IgG werden überraschend gute Ergebnisse erzielt.

Es ist für die Ausführung der erfindungsgemäßen Lehre bevorzugt, wenn z. B. die zu untersuchenden rekombinanten Proteine immobilisiert vorliegen. Diese Immobilisierung kann beispielsweise mithilfe von Lektinen erfolgen. Es ist völlig überraschend, dass die Verwendung von Lektinen zur Immobilisierung insbesondere der rekombinanten Proteine zu einer Verbesserung der Bestimmung der Zuckerstrukturen führt. Selbstverständlich ist es auch möglich, statt deglykosylierter Enzyme, die beispielsweise im Zusammenhang mit einer Avidin/Streptavidin/Biotin-Nachweisreaktion eingesetzt werden, fluoreszenzmarkierte Marker zu verwenden.

Wenn Enzyme in einem Avidin/Streptavidin-Biotin-Nachweissystem eingesetzt werden, so sind beispielsweise das Biotin, das Streptavidin und z. B. die alkalische Phosphatase (AP) deglykosyliert. In weiteren bevorzugten Ausführungsformen können neben der alkalischen Phosphatase auch die Horseradish-Peroxidase, die Beta-Galactosidase oder eine Urease verwendet werden.

In einer bevorzugten Ausführungsform der Erfindung wird demgemäß zunächst ein Lektin auf einer ELISA-Platte vorgelegt. Dieses dient der Erkennung der Glykostruktur der zu untersuchenden Probe. Zur Detektion der gebundenen Probe wird im weiteren Assay-Aufbau beispielsweise ein Sekundärantikörper eingesetzt. Bei diesem handelt es sich im Sinne der Erfindung um den deglykosylierten Detektor, also nur ein spezifisches Bindemolekül oder eine spezifische Bindestruktur, der/die in einer bevorzugten Ausführungsform mit Biotin/Vitamin H markiert sein kann. Der deglykosylierte Detektor wie der Sekundärantikörper oder ein Teil davon bindet spezifisch an die Probe. Zum Auslesen bzw. zum Nachweis der gebundenen Sekundärantikörper wird insbesondere ein Enzym eingesetzt. Das Enzym wird über das Streptavidin-Biotin-System an den Sekundärantikörper gebunden. Grundsätzlich kann auch eine direkte chemische Anbindung des Enzyms an den Sekundärantikörper erfolgen. Das Enzym ist beispielsweise eine alkalische Phosphatase oder aber eine Horseradish-Peroxidase, Beta-Galactosidase oder Urease. Die Enzyme zur Markierung liegen ebenfalls deglykosyliert vor. Demgemäß umfasst das deglykosylierte Detektionssystem in der beschriebenen bevorzugten Ausführungsform einen deglykosylierten Detektor wie den Sekundärantikörper und ein deglykosyliertes Enzym wie die alkalische Phosphatase. Das beschriebene System umfasst weiterhin Biotin und Streptavidin, wobei das Streptavidin bevorzugt keine Glykane aufweist. Die nicht glykosylierte alkalische Phosphatase ist kommerziell erhältlich und stammt aus Escherichia coli (entweder rekombinant oder nicht rekombinant). Dieses deglykosylierte Enzym zeigt in der Praxis aber keine besonders gute Aktivität gegenüber anderen glykosylierten Enzymen, die zum Vergleich eingesetzt werden können. Erfahrungsgemäß sinkt die Enzymaktivität durch eine weitere Behandlung wie z. B. durch die Kopplung an Streptavidin. Die besonders bevorzugte erfindungsgemäße Bereitstellung von aktiven Enzymen wie der alkalischen Phosphatase wird im Zusammenhang mit einer weiteren bevorzugten Ausführungsform der Erfindung erläutert.

Für den Fachmann mit durchschnittlichem Können sind die einzelnen technischen Merkmale der erfindungsgemäßen Lehre ausreichend klar definiert. Ihm ist aus der ELISA-Technik bekannt, dass er rekombinante Proteine, bevorzugt in Form von Antikörpern, sehr gut und sicher mit Detektoren nachweisen kann, wenn diese Detektoren spezifisch mit den bevorzugten Antikörpern reagieren. Es kann sich bei den Detektoren um Lektine, aber insbesondere um sekundäre Antikörper oder Fragmente hiervon handeln.

Dem Fachmann sind Detektionsmethoden für die Sichtbarmachung von bevorzugt primären Antikörpern bekannt. Mit Vorteil werden für den Nachweis der primären Antikörper insbesondere sekundäre Antikörper eingesetzt. Selbstverständlich ist es möglich, dass der Fachmann andere spezifische Detektoren für die primären Antikörper einsetzt, wie beispielsweise Protein A oder G. Protein A ist im Sinne der Erfindung ein Protein von 40 bis 60 kDa, das ursprünglich aus der Zellwand des Bakteriums Staphylococcus aureus stammt. Protein A wird vorteilhafterweise eingesetzt, um Immunoglobuline (primäre Antikörper) zu binden. Es werden Proteine verschiedener Säugerarten gebunden, vor allem IgG. Protein A bindet an die Fc-Region der Immunoglobuline durch Interaktion mit der schweren Kette. Insbesondere bindet Protein A mit hoher Affinität an humanes IgG1 und IgG2 sowie IgG2a und IgG2b. Aber auch IgM, IgA und IgG und rekombinante monoklonale Antikörper aus nicht-humanen Expressionssystemen werden spezifisch gebunden. Wenn der Fachmann vor die Aufgabe gestellt ist, primäre Antikörper zu binden, so weiß er, dass er neben sekundären Antikörpern, Protein A und/oder Fragmenten hiervon auch andere Detektoren verwenden kann, wobei Detektoren im Sinne der Erfindung Moleküle sind, die mit den primären Antikörpern spezifisch interagieren. Bei dem Detektor im Sinne der Erfindung kann es sich daher bevorzugt auch um Protein G handeln. Besonders spezifisch reagiert Protein G mit Ig1, Ig2 und Ig4 von humanem IgG; weiterhin bindet Protein G auch an Ig3 und weitere therapeutische Proteine. Überraschend ist es, dass Zuckerstrukturen von bevorzugt primären Antikörpern mit Detektoren sehr gut bestimmt werden können, wenn die Detektoren, also die Bindestrukturen, deglykosyliert vorliegen. Die Deglykosylierung kann vorteilhafterweise mithilfe von Glykosidasen, wie den Endo- und Exoglycosidasen erfolgen. Hier stehen dem Fachmann im Stand der Technik eine Reihe von Enzymen zur Verfügung. Im Zusammenhang mit der Erfindung zeigen Enzyme wie z.B. die N-Glykanasen, O-Glykanasen, Endoglykosidasen-H, F1, F2, F3, und die Sialidasen, Galaktosidasen, Mannosidasen und Fukosidasen ein überraschend gutes Resultat. Vorteilhafte Methoden der chemischen Deglykosylierung sind die Hydrazinolyse oder milde Säurehydrolyse zur Abspaltung von Sialinsäuren.

Neben den sekundären Antikörpern oder Protein A und/oder G, die zum Nachweis der primären Antikörper/therapeutischen oder diagnostischen Proteine eingesetzt werden können, ist es auch möglich, Fragmente dieser einzusetzen. Der Fachmann kann, ohne selbst erfinderisch tätig zu werden, durch einfache Routineversuche eruieren, ob ein Fragment eines sekundären Antikörpers oder von Protein A oder G effektiv eingesetzt werden kann, um Zuckerstrukturen von rekombinanten Proteinen, bevorzugt Antikörpern zu bestimmen.

Die Bestimmung der Zuckerstrukturen erfolgt bevorzugt mithilfe einer Enzym-Substrat-Reaktion, wobei - wie bereits ausgeführt - das Enzym neben dem eingesetzten Detektor ebenfalls deglykosyliert ist und diese beiden Strukturen das erfindungsgemäße Detektionssystem mit charakterisieren. Enzym-Substrat-Reaktionen sind bekannte Verfahren zum Nachweis von Antikörpern. Enzym-Substrat-Reaktionen sind häufiger Bestandteil der indirekten Methode des Nachweises eines spezifischen Antikörpers (Primärantikörpers). Ein bekanntes Verfahren der Enzym-Substrat-Reaktion sieht vor, dass ein Sekundärantikörper mit einem Enzym gekoppelt vorliegt, welches mit einem Substrat/Chromogen eine Farbreaktion initiiert, die durch Messgeräte oder durch einfaches Betrachten detektiert werden kann. Vorteilhaft eingesetzte Enzyme sind die Meerrettich-Peroxidase mit den Substraten ABTS, OPD oder TMB, die bovine alkalische Phosphatase mit den Substraten PNPP, FDP, NBT, oder auch die Enzyme beta-Galaktosidase und Glucose Oxidase mit entsprechenden Substraten. Bei der Verwendung der alkalischen Phosphatase werden als Substrate Phosphatverbindungen eingesetzt, von denen die alkalische Phosphatase Phosphat abspaltet, wodurch die freigesetzte Verbindung zu einem farbigen Endprodukt reagieren kann. Beispielsweise liefert Neufuchsin ein rosa/rotes Reaktionsprodukt, Naphthol-HS-MX-Phosphat bildet einen roten Farbstoff und 5-Brom-4-chlor-3-indoxylphosphat wird in Verbindungen mit Nitroblau-Tetrazoliumchlorid zu einem violetten bis blauen Farbstoff umgesetzt. Para-Nitrophenylphosphat (PNPP) wird zu einem gelben Farbstoff umgesetzt, der bei 405 nm detektiert werden kann, und Fluorescein-Diphosphat (FDP) wird nach Umsetzung zu Fluorescein mittels Fluoreszenzmessung detektiert.

In einer besonders bevorzugten Ausführungsform der Erfindung erfolgt die Deglykosylierung durch Abspaltung der Zucker vom Enzym, die Abtrennung glykosylierter Molekülteile und/oder die Herstellung des Enzyms in einem bakteriellen Expressionssystem. Die Deglykosylierung der Bindestrukturen oder des Detektors erfolgt durch Abspaltung der Zucker von den Bindestrukturen durch Abtrennung glykosylierter Molekülteile und/oder durch die rekombinante Herstellung mindestens eines Teils bzw. der vollständigen Bindestruktur. Der Begriff des Detektors und der Bindestrukturen kann synonym verwendet werden.

In einer besonders bevorzugten Ausführungsform erfolgt die Deglykosylierung bevorzugt des Markerenzyms durch mindestens drei Endoglykosidasen. Die Deglykosylierung erfolgt bevorzugt an immobilisierten Enzymen bzw. Markern. Die Verwendung einer Festphase ist vorteilhaft, da keine Nachreinigung der Enzyme wie der alkalischen Phosphatase erfolgen muss, was wiederum die Aktivität beeinflussen bzw. einen hohen zeitlichen Aufwand bedeuten würde, der durch die Immobilisierung vermieden werden kann. Die bevorzugten Enzyme sind Endoglykosidasen, insbesondere F1, F2 und F3 (Endo--N-acetylglucosaminidase F1 (F2, F3), bzw. Endo F1 (F2, F3)), die bevorzugt nacheinander in der genannten Reihenfolge verwendet werden. Bevorzugt werden diese aus Elizabethkingia meningosepticum (Chrysobacterium meningosepticum oder Flavobacterium meningosepticum) hergestellt oder sie werden rekombinant in E. coli exprimiert. Die Kopplung dieser Enzyme an Festphasen führt vorteilhafterweise und überraschenderweise zu nahezu keinem Verlust an Aktivität und es wird gleichzeitig überraschend eine vollständige Deglykosylierung erreicht.

Die Verwendung der bevorzugten Endoglykosidasen F1, F2 und F3 ist besonders vorteilhaft, wenn die Enzyme bevorzugt separat chemisch auf Trägermaterial gebunden werden. Das bevorzugte Trägermaterial zur chemischen Kopplung der Enzyme basiert auf einem Methacrylat. Die Enzyme werden bevorzugt separat an separate Trägermaterialien gekoppelt. Die Verwendung des so gefertigten Trägermaterials erfolgt bevorzugt ebenfalls in drei separaten Gefäßen (z. B. Kartuschen). In dieser besonders bevorzugte Ausführungsform erfolgt die Deglykosylierung also nacheinander. Selbstverständlich ist es auch möglich das Trägermaterial zu kombinieren, bspw. ein Trägermaterial an welchem F1 und F3 gebunden sind. In diesem Falle würde das Trägermaterial mit F2 in ein zweites separates Gefäß eingebracht werden. Die vorteilhafte Ausführungsform ist jedoch die Verwendung der gekoppelten Enzyme an Trägermaterial in drei separaten Gefäßen.

Bevorzugt wird die alkalische Phosphatase an den genannten drei Trägern glykosyliert, wobei die Qualifizierung im beschriebenen erfindungsgemäßen Glykanarray und im Enzymaktivitätstest erfolgt. Es ist auch möglich, die drei Endoglykosidasen auf einem Träger zu immobilisieren. Die alkalische Phosphatase kann aus verschiedenen Ursprungsorganismen gewonnen werden wie z. B. caff intestine, Bovineserum, Bovine (rekombinant aus Pichia pastoris), E. coli und als Referenz ein Streptavidin-AP-Konjugat verwendet wird, wobei die AP (bovine) glykosyliert ist. Die alkalische Phosphatase ist ein Enzym, welches sich sehr gut im Bereich der ELISA-Technik anwenden lässt. Aber auch die weiteren genannten Enzyme wie Horseradish-Peroxidase, Beta-Galactosidase oder die Urease sind gut einsetzbar. Die Enzyme können in ihrer nativen Form oder als Konjugate eingesetzt werden. Durch Routineversuche kann der Fachmann problemlos feststellen, welches Konjugat in Abhängigkeit vom jeweiligen ELISA verwendet werden soll. Beispielsweise können die Enzyme an Antikörper, deren Fragmente, an Avidin oder an Streptavidin oder an Lektine gebunden vorliegen. Streptavidin ist ein deglykosyliertes Komplement zum Avidin. Es zeigt trotz Fehlens der Glykane überraschenderweise kaum bzw. keine Unterschiede zum Avidin. Das Streptavidin wird bevorzugt aus dem Bakterium Streptomyces avidinii gewonnen. Es war überraschend, dass die Deglykosylierung aller Moleküle, die erfindungsgemäß das Detektionssystem darstellen, zu einem überraschend verbesserten Verfahren führen. Es ist für eine besonders gute Bestimmung im Sinne der Erfindung nicht ausreichend, wenn nur bestimmte Zuckergruppen an bestimmten Molekülen des Detektionssystems entfernt werden. Bei glykosylierten Detektoren oder Bindestrukturen wäre zum einen ein Signal auch dann detektierbar, wenn die Probe bindet, aber auch wenn die Probe nicht bindet, aber der Sekundärantikörper bindet, oder aber wenn die Probe nicht bindet, der Sekundärantikörper nicht bindet, aber das Enzym bindet, wodurch bei der Messung nicht sicher bestimmt werden kann, auf wen das Signal zurückzuführen ist. Damit wird beispielsweise bei einem Lektinarray ein Mischsignal erzeugt, aus welchem nicht abgelesen werden kann, welches Signal tatsächlich durch die zu untersuchende Probe hervorgerufen wurde.

Die erfindungsgemäße Verwendung der deglykosylierten Detektoren/Bindestrukturen - bevorzugt sekundäre Antikörper - und den Markerenzymen erfolgt insbesondere in einem Verfahren zur Bestimmung der Zuckerstrukturen von rekombinanten Proteinen, insbesondere Immunoglobulinen, welches die folgenden Schritte umfasst:
a) Bereitstellung des zu untersuchenden rekombinanten Proteins, insbesondere eines rekombinanten therapeutischen Glykoproteins, wobei das Protein immobilisiert vorliegt, bevorzugt mittels eines deglykosylierten Lektins;
b) Inkubation mit einer markierten Bindestruktur, bevorzugt einem sekundären Antikörper oder einem Protein A oder Fragment davon, welcher deglykosyliert ist; und
c) Bestimmung der Interaktion zwischen dem Protein und der Bindestruktur.

In diesem Verfahren können insbesondere Immunoglobuline, ausgewählt aus der Gruppe IgG, IgM, IgE, IgD, IgA, IgY und/oder IgW in Bezug auf ihre Zuckerstrukturen untersucht werden. D. h. die erfindungsgemäße Verwendung von deglykosylierten Bindestrukturen (Detektoren) und Markerenzymen bzw. Enzymen ermöglicht effiziente, sichere, schnelle und automatisierbare Verfahren zur Bestimmung der Zuckerstrukturen von Proteinen, insbesondere Immunoglobulinen.

Bei dem Verfahren kann bevorzugt zunächst ein Array von Lektinen auf eine Mikrotiterplatte aufgebracht werden, die Zuckerstrukturen bevorzugt am IgG erkennen. Lektine sind im Stand der Technik als Proteine bekannt, die ein oder mehrere Bindungsstellen für Zuckerstrukturen aufweisen. Lektine werden daher eingesetzt, um Zuckerstrukturen zu detektieren oder abzutrennen. Die Lektine können native Lektine sein ausgewählt aus den Organismen Agaricus bisporus, Aleuria aurantia, Amaranthus caudatus, Anguilla anguilla, Arachis hypogaea, Artocarpus integrifolia, Griffonia (Bandeiraea) simplicifolia 1, Bauhinia purpurea alba, Cicer arietinum, Codium fragile, Canavalia ensiformis, Datura stramonium, Dolichos biflorus, Erythrina coralldendron, Erythrina cristagalli, Euonymos europaeus, Galanthus nivalis, Glycine max, Helix aspersa, Helix pomatia, Hippeastrum hybrid, Lens culinaris, Limulus polyphemus, Lotus tetragonolobus, Lycopersicon esculentum, Maackia amurensis, Maclura pomifera, Narcissus pseudonarcissus, Phaseolus coccineus, Phaseolus limensis, Phaseolus vulgaris, Phytolacca americana, Pisum sativum, Phosphocarpus tetragonolobus, Ricinus communis, Sambucus nigra, Solanum tuberosum, Sophora japonica, Triticum vulgaris, Tritrichomonas mobilensis (Protozoe), Ulex europaeus, Vicia faba, Vicia villosa, Viscum album, Wisteria floribunda und/oder auch rekombinante Lektine. In einer Ausführungsform des Verfahrens werden unterschiedliche Lektine (mindestens eines) auf der Oberfläche einer Mikrotiterplatte immobilisiert. Die Lektine werden so gewählt, dass sie die Zuckerstrukturen bevorzugt am IgG erkennen, insbesondere N-Acetylglucosamin, Galaktose, Mannose, Fukose und Sialinsäure (N-Acetlyneuraminsäure). Die Erkennung einer spezifischen Zuckerstruktur bevorzugt am IgG führt dazu, dass das IgG Molekül an der Oberfläche zurückgehalten wird, während andere Moleküle, die diese Struktur nicht enthalten, abgewaschen werden.

Als bevorzugte Kontrolle für die IgGs kann Protein A oder Fragmente dessen eingesetzt werden. Protein A bindet an einem nicht-glykosylierten Teil des IgG (Fc) und stellt daher ein Referenzsignal zur Verfügung, das im Verhältnis zur Gesamtmenge des im Verfahren eingesetzten IgG steht. Das Protein A Signal kann also zur Kontrolle des Verfahrens und zur Kalibrierung verwendet werden, um aus dem Verfahren auch quantitative Informationen zu erhalten. Die Einzelergebnisse für die Zuckerstrukturen können im Vergleich zu der Protein A-Kontrolle auf den IgG-Gesamtgehalt bezogen werden.

Nachdem in der bevorzugten Ausführungsform der Array von Lektinen auf einer Mikrotiterplatte bereitgestellt wurde, kann dieser Array mit der Probe, die die zu untersuchenden Glykoproteine umfasst, in Kontakt gebracht werden. Hierbei sind die zu untersuchenden Glykoproteine insbesondere rekombinante Antikörper; die als primäre Antikörper bezeichnet werden. Primäre Antikörper sind die Moleküle, deren Zuckerstruktur bestimmt wird, indem sie bevorzugt mit einem zweiten Antikörper (= sekundärer Antikörper) detektiert werden. Vorteilhafterweise kann die Probe ohne weitere Vorbereitung, wie beispielsweise einer Markierung der in ihr enthaltenen Glykoproteine, eingesetzt werden. Im Stand der Technik sind aufwändige Vorbereitungen der Probe erforderlich.

Nach diesem Verfahrensschritt liegt ein Array von Lektinen auf einer Mikrotiterplatte vor, an dem die spezifisch bindenden, zu untersuchenden Glykoproteine gebunden vorliegen.

Die Interaktion zwischen dem zu untersuchenden Protein und dem Lektin kann beispielsweise mit einer dem Fachmann bekannten Enzym-Substrat-Reaktion bestimmt werden, wie sie dem Fachmann insbesondere von ELISA-Tests bekannt ist. Erfindungsgemäß kann mit dem Begriff des Enzyms - z. B. im Zusammenhang mit einer Enzym-Substrat-Reaktion - das Markerenzym gemeint sein, d. h. das Enzym, welches zur Detektion verwendet wird. Auch die Strukturen, um dieses Markerenzym bzw. Enzym zu deglykosylieren, sind biochemisch wiederum Enzyme, beispielsweise Endoglykosidasen, bevorzugt F1, F2 und F3, die getrennt auf Trägern aufgebaut in der genannten Reihenfolge nacheinander zur Deglykosylierung des Markerenzyms oder des Detektors eingesetzt werden können. Der Fachmann erkennt aus der Gesamtoffenbarung, wann mit dem Begriff des Enzyms ein Molekül zur Detektion (Markerenzym) gemeint ist und wann ein Molekül, welches zur Deglykosylierung eingesetzt wird.

Im Sinne der Erfindung wird die Interaktion mit einem so genannten Detektor oder einer Bindestruktur bestimmt, indem der Detektor/die Bindestruktur das zu untersuchende Glykoprotein in der Probe spezifisch erkennt und zwar besonders bevorzugt an einer Stelle, die nicht das Glykan ist. Der Detektor bzw. das Detektormolekül oder der Binder oder die Bindestruktur ist insbesondere ein sekundärer Antikörper oder ein Fragment dessen, es kann sich aber auch um ein Protein A oder ein Fragment hiervon handeln. Demgemäß kann der Begriff Detektor oder Bindestruktur oder aber Binder oder Detektionsmolekül oder Detektormolekül im Sinne der Erfindung synonym verwendet werden. Der durchschnittliche Fachmann erkennt, dass der erfindungsgemäße deglykosyliert vorliegende Detektor bevorzugt ein Sekundärantikörper oder ein Teil hiervon ist oder Protein A oder G bzw. ein Fragment hiervon, welches ein wesentlicher Bestandteil des erfindungsgemäßen deglykosylierten Detektionssystems ist. Wenn neben dem deglykosylierten Detektor bzw. der deglykosylierten Bindestruktur noch das deglykosylierte Markerenzym vorliegt und der deglykosylierte Detektor beispielsweise Biotin aufweist und das deglykosylierte Markerenzym beispielsweise Streptavidin, so stellt diese genannte Kombination das bevorzugte Detektionssystem gemäß der Erfindung dar, welches ein deglykosyliertes Detektionssystem ist.

Es ist erfindungswesentlich, dass diese Detektormoleküle deglykosyliert vorliegen, um falsch-positive Hintergrundsignale ausschließen zu können. Experimente haben gezeigt, dass man bei Verwendung von glykosylierten Detektoren ein Mischergebnis erhält, dass sowohl von den Zuckerstrukturen des Detektors als auch von denen des zu analysierenden Proteins bestimmt wird. Das Anwendungsbeispiel 1 zeigt die Signale eines glykosylierten Detektors auf 2 unterschiedlichen Lektinen.

Überraschenderweise führt die zusätzliche Deglykosylierung des Enzyms, welches bei der Enzym-Substrat-Reaktion verwendet wird, also des Markerenzyms, zu einem so überraschend guten Ausschluss der falsch-positiven Hintergrundsignale, dass eine einfache, sichere und effiziente Bestimmung der Zuckerstrukturen der rekombinanten therapeutischen Proteine möglich ist. Insbesondere therapeutische einzusehende Proteine wie Antikörper können ganz besonders sicher und effizient mit dem erfindungsgemäßen Verfahren bestimmt werden. Experimente haben gezeigt, dass man bei Verwendung von glykosylierten Enzymen, d. h. den Markerenzymen ein Mischergebnis erhält, dass sowohl von den Zuckerstrukturen des Enzyms als auch von denen des zu analysierenden Proteins bestimmt wird. Das Anwendungsbeispiel 2 zeigt den Vergleich eines glykosylierten und eines nicht-glykosylierten Enzym auf einer lektinbeschichteten Mikrotiterplatte.

In einer bevorzugten Ausführungsform der Erfindung erfolgt die Bestimmung der Interaktion zwischen dem Protein und dem Detektor über eine Enzym-Substrat-Reaktion.

In einer weiteren besonders bevorzugten Ausführungsform der Erfindung liegt das rekombinante Protein immobilisiert vor. Bei der Immobilisierung handelt es sich im Sinne der Erfindung um verschiedene Verfahren zum Fixieren der rekombinanten Proteine, insbesondere der Antikörper, insbesondere von IgG. Die Fixierung insbesondere der Antikörper kann durch biologische, chemische oder physikalische Einwirkung erfolgen. Beispielsweise ist es möglich, die rekombinanten Proteine/Antikörper trägergebunden zu fixieren, oder aber durch Quervernetzung, durch Einschluss oder durch das Einbringen in Mikrokapseln. Bei der Bindung an einen Träger (z. B. Mikrotiterplatte) erfolgt die Fixierung durch Adsorption, Ionenbindung oder kovalente Bindung. Beispielsweise können die rekombinanten Proteine/Antikörper auch durch einen immobilisierten Sekundärantikörper (Fängerantikörper) oder Protein A an der Oberfläche fixiert werden. Bevorzugt wird das Protein durch die Fixierung in seiner Zuckerstruktur nicht nachteilhaft modifiziert. Bei dem Einschluss in eine semipermeable Membran erfolgt die Fixierung in Form des Einschlusses in Gele, Mikrokapseln oder Fasern.

Bevorzugt erfolgt die Fixierung des rekombinanten Proteins, insbesondere der Antikörper, bevorzugt von IgG, an eine Mikrotiterplatte. Bei Mikrotiterplatten handelt es sich bevorzugt um Plastikplatten in der Regel aus Polystyrolen oder Polyvinylchlorid, die für unterschiedliche immunologische Arbeitsgänge eingesetzt werden können. Die gängigen Mikrotiterplatten haben 96 Vertiefungen mit einem Fassungsvermögen von je 250 Mikroliter. Selbstverständlich ist es auch möglich, Mikrotiterplatten aus anderen Materialien einzusetzen oder aber die Anzahl der Vertiefungen zu variieren. Für den Hochdurchsatz sind beispielsweise auch Mikrotiterplatten mit 384 oder 1536 Vertiefungen vorteilhaft.

In einer besonders bevorzugten Ausführungsform der Erfindung wird das rekombinante Protein, insbesondere der Antikörper, bevorzugt IgG über ein Lektin fixiert/immobilisiert. Lektine im Sinne der Erfindung sind insbesondere komplexe Proteine, die spezifische Kohlenhydratstrukturen des Proteins, welches untersucht werden soll, binden und dadurch in der Lage sind, dieses zu fixieren. Lektine werden insbesondere aus Pflanzenarten, vor allem aus Hülsenfrüchten, aber auch aus tierischen Organismen und Zellen gewonnen. Bevorzugte Lektine haben eine Spezifität für Galaktose, Mannose, N-Acetylglukosamin, Fukose und Sialinsäuren (N-Acetylneuraminsäure) und werden beispielsweise aus der Schwertbohne, Gartenbohne, Ricinusbohne, Sojabohne, Linse, Erbse, Erdnuss, Weizen, Kartoffel, Schneeglöckchen, dem schwarzen Holunder, dem Amur-Gelbholz, dem Stechginster, dem Pfaffenhütchen, und/oder aus Pilzen wie dem Orangebecherling gewonnen.

In einer besonders bevorzugten Ausführungsform erfolgt die Immobilisierung des zu untersuchenden therapeutischen rekombinanten Glykoproteins, insbesondere des primären Antikörpers dergestalt, dass es über ein Lektin fixiert wird, welches an einer Mikrotiterplatte immobilisiert vorliegt. Dem Fachmann sind Kombinationen aus Mikrotiterplatte und einem hierauf immobilisierten Lektin aus anderen Verwendungen bekannt. Derartige Kombinationen sind beispielsweise aus Enzym-Immunoassays bekannt. Enzym-Immunoassays umfassen Enzyme und Substrate bzw. Radioisotope. Alle diese Begriffe sind im Stand der Technik übliche Begriffe und lexikalisch nachweisbar und müssen daher im Detail nicht erläutert werden; wie auch der Begriff des Detektors, der Bindestrukturen, Markenenzyms oder des Detektionssystems. Die Immobilisierung eines Lektins auf einer Mikrotiterplatte ist bevorzugter Bestandteil der bekannten Festphasentechnik, beispielsweise der Affinitätschromatographie.

Bevorzugt werden Lektine auf der Mikrotiterplatte immobilisiert, die mit einer Zuckerstruktur des rekombinanten Proteins, insbesondere der Antikörper, bevorzugt IgG spezifisch wechselwirken. Eine spezifische Wechselwirkung ist eine Wechselwirkung, die einen Rückschluss auf eine Zuckerstruktur des rekombinanten Proteins zulässt. Eine Wechselwirkung, die dergestalt erfolgt, dass die Zuckerstruktur nicht bestimmt werden kann, ist nicht spezifisch im Sinne der Erfindung. Eine nicht spezifische Wechselwirkung wäre beispielsweise eine Wechselwirkung eines Mittels mit der Mikrotiterplatte, mit dem Lektin und/oder dem rekombinanten Protein, insbesondere seiner Zuckerstruktur, so dass keine Bestimmung der Zuckerstruktur möglich ist. Dieser Aufbau des Verfahrens führt zu besonders guten Ergebnissen.

Besonders bevorzugt ist es, dass in vorteilhaften Ausführungsformen der Erfindung das rekombinante Protein/Glykoprotein ohne eine Markierung eingesetzt wird. D. h., vorteilhafterweise ist es nicht erforderlich, dass die Probe, die das zu untersuchende Glykoprotein/rekombinante Protein, bevorzugt den Antikörper, insbesondere IgG umfasst, vorbereitet wird, beispielsweise indem eine Markierung mit Enzymen, Biotin/Streptavidin, Fluoreszenzfarbstoffen wie FITC oder Isotopen erfolgt. Selbstverständlich kann aber - wie oben ausgeführt - eine Markierung der Markerenzyme mit spezielleren weiteren Enzymen oder aber Fluoreszenzfarbstoffen oder Isotopen erfolgen.

In einer besonders bevorzugten Ausführungsform umfasst das erfindungsgemäße Verfahren die folgenden Schritte:
a) Bindung mindestens eines Lektins auf einer Mikrotiterplatte;
b) In-Kontakt-Bringen des Lektins mit dem zu untersuchenden rekombinanten Protein/Glykoprotein;
c) Inkubieren mit einem/einer markierten Detektor/Bindestruktur, umfassend einen sekundären Antikörper, Protein A und/oder Fragmenten dieser;
d) Nachweis des/der Detektors/Bindestruktur mit Hilfe einer Enzym-Substrat-Reaktion, wobei der/die Detektor/Bindestruktur und das Enzym deglykosyliert sind.

In diesem Falle erkennt der Fachmann, dass mit dem Enzym das Markerenzym gemeint ist, welches deglykosyliert, bevorzugt mit Streptavidin, welches keine Glykane besitzt, vorliegt. Es war überraschend, dass mit dem Verfahren eine besonders effiziente Bestimmung der Zuckerstrukturen möglich ist.

Selbstverständlich ist es auch möglich, das erfindungsgemäße Verfahren wie folgt zu modifizieren, so dass es folgende chronologische Schrittabfolge umfasst:
a) Bindung mindestens eines deglykosylierten Detektors bevorzugt eines sekundären Antikörpers auf einer Mikrotiterplatte;
b) In-Kontakt-Bringen des Detektors mit dem zu untersuchenden Protein/Glykoprotein;
c) Inkubieren mit einem markierten Lektin;
d) Nachweis des Detektors mit Hilfe einer Enzym-Substrat-Reaktion, wobei der Detektor und das Enzym deglykosyliert sind.

Auch bei diesem bevorzugten Verfahren erkennt der Fachmann, dass mit dem Enzym das Markerenzym gemeint ist und mit dem Detektor die erfindungsgemäße Bindestruktur, da die Bindestruktur und der Detektor im Sinne der Erfindung synonym verwendet werden können. In beiden Fällen ist jeweils bevorzugt ein deglykosyliertes Molekül wie beispielsweise ein Sekundärantikörper, Protein A oder G bzw. ein Fragment hiervon gemeint.

Besonders bevorzugt ist die vollständige Deglykosylierung im Zusammenhang mit den erfindungsgemäßen Verwendungen des deglykosylierten Detektors bzw. des Enzyms oder der Fragmente. Die vollständige Deglykosylierung des Detektors bzw. dessen Fragmentes wird z. B. durch die enzymatische Spaltung des Detektors erreicht. Anschließend wird das nicht glykosylierte Fab-Fragment oder Fab-2-Fragment verwendet, wenn der Detektor ein sekundärer Antikörper ist. Nichtglykosylierte Fab-Fragmente erhält man entweder durch Auswahl aus nativen, polyklonalen Formen oder durch die rekombinante, monoklonale Herstellung eines sekundären Antikörpers oder Fab-Fragmentes nach Ausschalten der möglichen Glykosylierungsstellen am Fab-Teil. Dem Fachmann sind diese Methoden bekannt. Bevorzugte Enyzme für die enzymatische Spaltung des Detektors, insbesondere des sekundären Antikörpers sind Papain und Pepsin. Weiterhin ist es vorteilhaft, eine vollständige oder teilweise Deglykosylierung des Detektors mit einer Behandlung dessen mit zuckerabspaltenden Enzymen, bevorzugt Glykosidasen vorzunehmen. Hierzu können bevorzugt folgende Enzyme verwendet werden: Endo F1, Endo F2, Endo F3, PNGase F, N-Glycanase, Galaktosidase, Mannosidase, Sialidase und/oder Fukosidase. Auf diese Weise können sowohl der vollständige Detektor/Sekundärantikörper oder die Fragmente dessen (Fc, Fab, Fab-2) behandelt, d. h. deglykosyliert werden. Eine weitere bevorzugte Variante der Deglykosylierung ist eine chemische Behandlung, beispielsweise mit Periodat. Es kann weiterhin vorteilhaft sein, den Sekundärantikörper aus Expressionssystemen zu verwenden, die nur definierte Zuckerstrukturen synthetisieren, wie z. B. high-mannose-type, in Hefezellen oder ausgewählten, modifizierten eukaryotischen Systemen. Eine weitere bevorzugte Variante ist die Verwendung eines Detektors (z.B. Protein A) aus Expressionssystemen, die nicht zur post-translationalen Modifikation fähig sind, wie z. B. prokaryotische Systeme.

Bei der Bereitstellung des deglykosylierten Enzyms ist es bevorzugt möglich, dieses rekombinant oder nativ in prokaryotischen Expressionssystemen herzustellen, so dass es nicht glykosyliert ist. Dies ist beispielsweise besonders bevorzugt mit der nativen alkalischen Phosphatase oder der rekombinanten alkalischen Phosphatase aus E. coli möglich. Die bevorzugte vollständige enzymatische Deglykosylierung des Enzyms kann außerdem mit folgenden Enzymen erfolgen: Endo F1, Endo F2, Endo F3 (auch als Endo H F1, Endo H F2, Endo H F3 beschrieben), N-Glycanase, Galaktosidase, Mannosidase, Sialidase und/oder Fukosidase. Weiterhin ist die Verwendung eines Enzyms aus Expressionssystemen möglich, die nur definierte Zuckerstrukturen synthetisieren (z. B. high-mannose-type, in Hefezellen oder andere ausgewählte eukaryotische Systeme).

In einer weiteren bevorzugten Ausführungsform der Erfindung wird das rekombinante Protein über ein Lektin immobilisiert.

In einer weiteren bevorzugten Ausführungsform der Erfindung liegt das Lektin auf einer Mikrotiterplatte immobilisiert vor.

In einer weiteren bevorzugten Ausführungsform der Erfindung sind die Lektine so ausgewählt, dass sie mit einer Zuckerstruktur des rekombinanten Proteins spezifisch wechselwirken.

Die erfindungsgemäße Lehre stellt ein Verfahren zur besonders effizienten und störungsfreien Bestimmung von Zuckerstrukturen von rekombinanten Proteinen zur Verfügung. Es war insbesondere völlig überraschend, dass insbesondere immobilisierte, rekombinante Proteine mit einem deglykosylierten Detektionssystem im Hinblick auf Ihre Zuckerstrukturen sehr gut untersucht werden können, wenn das deglykosylierte Detektionssystem ein deglykosyliertes Markerenzym, bevorzugt zur Durchführung einer Enzym-Substrat-Reaktion umfasst und eine deglykosylierte Bindestruktur, bevorzugt einen sekundären Antikörper. Ein derartiges Verfahren ist im Stand der Technik nicht bekannt und wird durch diesen auch nicht nahegelegt. In Lundy et al., Molecular Biotechnology, Band 12, Nr. 2, S. 203-206, September 1999 wird behauptet, dass der dort vorgestellte Assay an jede Aufgabenstellung angepasst werden kann. In dieser Druckschrift wird der Antikörper deglykosyliert, was eine Anpassung an alle weiteren Assays zur Bestimmung von Glykoproteinen erlauben soll. Demgemäß ist der Fachmann nicht motiviert, im Lichte dieser Druckschrift ein deglykosyliertes Detektionssystem wie das erfindungsgemäße bereitzustellen, da die genannte Druckschrift dem Fachmann die Lehre vermittelt, dass das Hintergrundrauschen durch die Deglykosylierung der Antikörper auf einen Wert reduziert werden kann, dass eine optimale Bestimmung von Glykoproteinen möglich ist. Es zeigt sich aber in Versuchen der Erfinder, dass das Hintergrundrauschen nicht optimal vermieden werden kann und dass auch keine gute Sensitivität gewährleistet werden kann, zumal in der genannten Offenbarung die Problematik in der Sensitivität des Nachweises nicht diskutiert wird. Auch die beschriebene Abtrennung von Glykanen über die PNGase F ist nicht ausreichend, um einen ausreichend sensitiven Assay bereitzustellen. Da das Enzym ALP (AP) gemäß der genannten Offenbarung glykosyliert vorliegt, kann das dort beschriebene Verfahren nicht für verschiedene Zielstrukturen eingesetzt werden. Insbesondere die Untersuchung von rekombinanten Proteinen wie Antikörpern erfordert eine besonders feine Abstimmung und Auswahl der Assay-Komponenten. Antikörper sind an Fc-Teil oft weniger glykosyliert und die Glykane sind für das Lektin sterisch nicht optimal zugänglich. Das in der genannten Offenbarung beschriebene Verfahren kann daher nicht zur Untersuchung von Antikörpern eingesetzt werden, da hier ein besonders hohes Maß an Sensitivität gefordert wird. Nur mit dem erfindungsgemäßen Verfahren wird die erforderliche Sensitivität erreicht. Ein Fachmann wird durch die Gesamtoffenbarung der genannten Druckschrift nicht motiviert, diese beispielsweise mit der EP 1 460 425 zu kombinieren. Bevorzugt wird nur durch die Deglykosylierung an einem Träger die völlige Freiheit von Glykostrukturen beim Markerenzym erreicht. Die bevorzugte Kombination aus den Glykosidasen F1, F2 und F3 führt zu einem ausreichend sensitiven Assay. Bevorzugt erfolgt die Deglykosylierung vor der Konjugation an beispielsweise Streptavidin. Diese bevorzugte Ausführungsform garantiert zum einen die Freiheit von Glykostrukturen in dem erfindungsgemäßen Detektionssystem und eine ausreichende Aktivität nach Konjugation. Bevorzugt ist die chemische Konjugation von Streptavidin an das Enzym.

Im Folgenden soll die Erfindung anhand von Beispielen näher erläutert werden, ohne auf dieses beschränkt zu sein (Anwendungsbeispiele 1 bis 3).

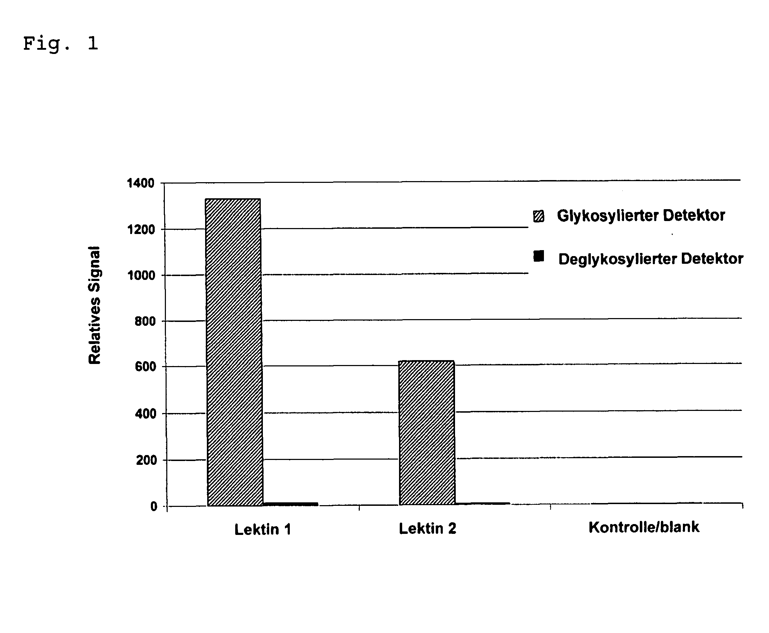

### Anwendungsbeispiel 1 (Fig. 1):

Die Oberfläche einer Mikrotiterplatte wurde mit den Lektinen Concanavalin A (1) und Sambucus nigra agglutinin (2) beschichtet. Um zu ermitteln, welches Signal von einem glykosylierten Detektor herrührt, wurde ein nativer Sekundärantikörper (aus Ziege) ohne Zugabe einer Probe direkt vermessen. Als Vergleich wurde ein deglykosylierter Detektor (ProteinA) vermessen.

Die Ergebnisse zeigen hohe Signale des glykosylierten Sekundärantikörpers (Detektors) auf beiden verwendeten Lektinen. Die Kontrolloberfläche (Blank = serum albumin) zeigt erwartungsgemäß keine Anbindung des glykosylierten Detektors. Der deglykosylierte Detektor Protein A liefert ebenfalls keine Signale auf den Lektinen.

Das Anwendungsbeispiel zeigt, dass nur die Verwendung eines deglykosylierten Detektors falsch-positive Hintergrundsignale ausschließen kann.

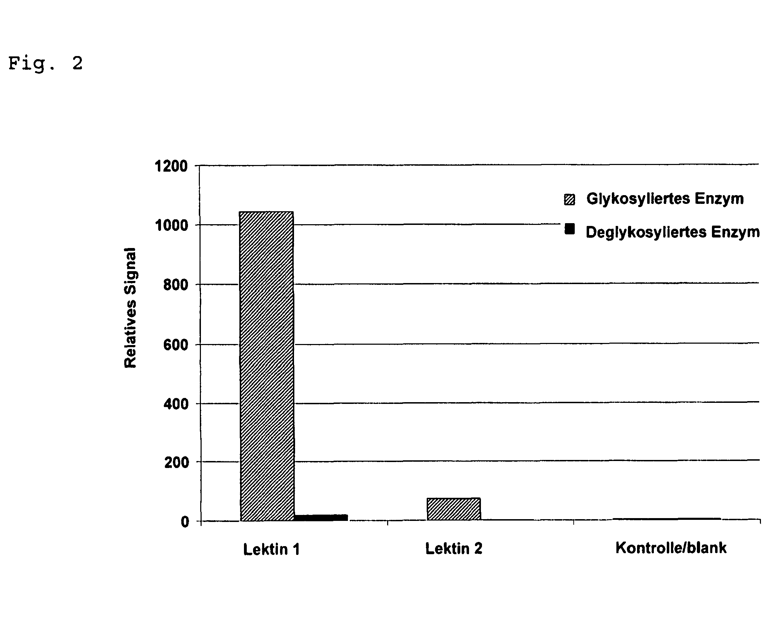

### Anwendungsbeispiel 2 (Fig. 2):

Die Oberfläche einer Mikrotiterplatte wurde mit den Lektinen Aleuria aurantia lektin (1) und Galanthus nivalis agglutinin (2), Protein A und Serumalbumin (blank) beschichtet. Um zu ermitteln, welches Signal von einem glykosylierten Enzym herrührt, wurden im Vergleich ein glykosyliertes und ein deglykosyliertes Enzym (hier alkalische Phosphatase) vermessen. Zur Verdeutlichung wurde in diesem Fall keine Probe, z.B. ein therapeutisches IgG, zugegeben.

Die Ergebnisse zeigen ein hohes Signal des glykosylierten Enzyms am Lektin, das deglykosylierte Enzym zeigt hingegen kein Signal. Die Kontrolloberflächen Protein A und Serumalbumin zeigen erwartungsgemäß keine Anbindung des glykosylierten und des deglykosylierten Enzyms.

Das Anwendungsbeispiel zeigt, dass nur die Verwendung eines deglykosylierten Enzyms die falsch-positiven Hintergrundsignale ausschließen kann.

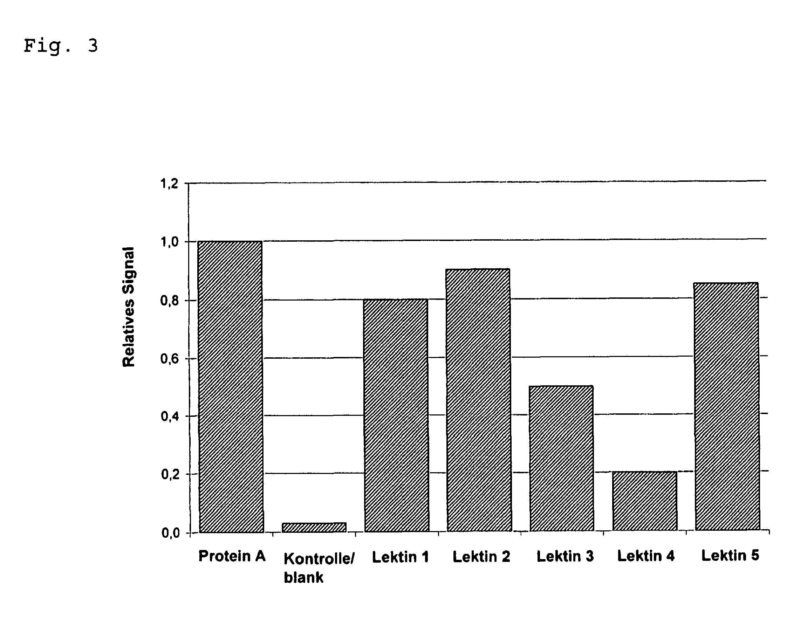

### Anwendungsbeispiel 3 (Fig. 3):

Die Oberfläche einer Mikrotiterplatte wurde mit einem Array von Lektinen (1-5), Protein A als Kontrolle und Serumalbumin (als blank) beschichtet. Die untersuchte Probe, ein rekombinantes, therapeutisches IgG, wurde anschließen aufgegeben und bindet entsprechend der vorhandenen Zuckerstrukturen an den Lektinen. Zur Detektion wurde ein biotinylierter Detektor (deglykosyliertes Fab-Fragment eines Sekundärantikörpers) eingesetzt, der nach einem Waschschritt mittels Spreptavidin-Alkalischer Phosphatase (deglykosyliert, rekombinant aus Ecoli) und dem Substrat Para-nitrophenylphosphat ausgelesen wird.

Die Ergebnisse zeigen unterschiedliche hohe Signale für die verschiedenen Lektine, die auf die speziellen Zuckerstrukturen der Probe zurückzuführen sind. Das ProteinA-Signal dient dabei der Normierung, damit unterschiedliche Proben direkt verglichen werden können. Das Anwendungsbeispiel zeigt, dass der erfindungsgemäße Assay geeignet ist, die Zuckerstrukturen von rekombinanten Proteinen schnell, automatisierbar und störungsfrei zu analysieren.

## Patentansprüche

1. Verwendung eines deglykosylierten Detektionssystems zur gleichzeitigen Bestimmung von mehrerer Zuckerstrukturen von rekombinanten Antikörpern, ausgewählt aus der Gruppe umfassend IgG, IgM, IgE, IgD, IgA, IgY und/oder IgW,
wobei das Detektionssystem ein deglykosyliertes Markerenzym und eine deglykosylierte Bindestruktur umfasst, und
die rekombinanten Antikörper mithilfe von Lektinen immobilisiert werden.

2. Verwendung nach dem vorhergehenden Anspruch,
**dadurch gekennzeichnet, dass**
die Bindestrukturen mindestens sekundäre Antikörper, Protein A und/oder Fragmente hiervon beinhalten.

3. Verwendung nach einem der vorhergehenden Ansprüche,
**dadurch gekennzeichnet**t, dass
die Bestimmung der Zuckerstrukturen mit Hilfe einer Markerenzym-Substrat-Reaktion erfolgt, wobei das Enzym deglykosyllert ist.

4. Verwendung nach einem der vorhergehenden Ansprüche,
**dadurch gekennzeichnet, dass**
das deglykosylierte Enzym aus der Gruppe umfassend alkalische Phosphatase, horseradish Peroxidase, β-Galactosidase und/oder Urease ausgewählt ist.

5. Verfahren zur Bestimmung von mehreren Zuckerstrukturen von rekombinanten Antikörpern, **dadurch gekennzeichnet, dass** das Verfahren folgende Schritte umfasst:
a) Bereitstellung des zu untersuchenden rekombinanten Antikörpers, wobei der Antikörper immobilisiert vorliegt, bevorzugt mittels eines deglykosylierten Lektins;
b) Inkubation mit einer deglykosylierten markierten Bindestruktur, bevorzugt einem sekundären Antikörper oder einem Protein A oder Fragment davon;
c) Bestimmung der Interaktion zwischen dem Antikörper und der Bindestruktur mit einer Markerenzym-Substrat-Reaktion, wobei das Enzym deglykosyliert ist; und
d) das Lektin auf einer Mikrotiterplatte immobilisiert vorliegt.

6. Verfahren nach Anspruch 5 **dadurch**
**gekennzeichnet, dass**
die rekombinanten Antikörper ohne eine Markierung eingesetzt werden.

7. Verfahren nach Anspruch 5 oder 6
**dadurch gekennzeichnet, dass**
das Verfahren die folgenden Schritte umfasst:
a) Bindung mindestens eines Lektins auf einer Mikrotiterplatte;
b) In-Kontakt-Bringen des Lektins mit dem zu untersuchenden rekombinanten Antikörper;
c) Inkubieren mit einer markierten Bindestruktur, umfassend mindestens einen sekundären Antikörper, Protein A, ein Markerenzym oder Fragmente dieser;
d) Nachweis der markierten Bindestruktur mit Hilfe einer Markerenzym-Substrat-Reaktion, wobei der sekundäre Antikörper, das Protein A, das Enzym oder Fragmente hiervon deglykosyliert sind.

8. Verwendung eines deglykosylierten Detektionssystems zur Bestimmung mehrerer Zuckerstrukturen von rekombinanten Antikörpern zur Durchführung eines Verfahrens nach einem der Ansprüche 5 bis 7,
**dadurch gekennzeichnet, dass**
das Detektionssystem ein deglykosyliertes Markerenzym und eine deglykosylierte Bindestruktur umfasst.

9. Verwendung eines Zuckerbestimmungskit zur Durchführung des Verfahrens gemäß einem der Ansprüche 5 bis 7, umfassend mindestens
- eine deglykosylierte Bindestruktur, ausgewählt aus der Gruppe umfassend Antikörper, Protein A oder Fragmente hiervon, und
- ein deglykosyliertes Enzym und
- gegebenenfalls eine Information zum Kombinieren der Inhalte des Kits.

## Claims

1. Use of a deglycosylated detection system for the simultaneous identification of multiple sugar structures of recombinant antibodies, selected from the group comprising IgG, IgE, IgD, IgA, IgY and/or IgW whereby the detection system comprises a deglycosylated marker enzyme and a deglycosylated binding structure and the recombinant antibodies are immobilized by means of lectins.

2. Use according to the previous claim, **characterized in that** the binding structures contain at least secondary antibodies, protein A and/or fragments thereof.

3. Use according to one of the previous claims, **characterized in that** the sugar structures are identified by means of a marker enzyme substrate reaction whereby the enzyme is deglycosylated.

4. Use according to one of the previous claims, **characterized in that** the deglycosylated enzyme is selected from the group comprising phosphatase, horseradish peroxidase, beta-galactosidase and/or Urease.

5. Process for the identification of multiple sugar structures of recombinant antibodies, **characterized in that** the process includes the following steps:
a) Preparation of the recombinant antibodies to be identified whereby the antibody is immobilized, preferably using deglycosylated lectins;
b) Incubation with a deglycosylated marked binding structure, preferably a secondary antibody or a protein A or a fragment thereof;
c) Identification of the interaction between the antibody and the binding structure with a marker enzyme substrate reaction whereas the enzyme is deglycosylated and
d) the lectin is immobilized on a microtitre plate.

6. Process according to claim 5, **characterized in that** the recombinant antibodies are used without marking.

7. Process according to claim or claim 6, **characterized in that** the process includes the following steps:
a) Binding of at least one lectin on microtitre plate;
b) Bringing the lectin into contact with the recombinant antibody to be examined;
c) Incubation with a marked binding structure, comprising at least one secondary antibody, protein A, a marker enzyme or fragments thereof;
d) Verification of the marked binding structure using a marker enzyme substrate reaction whereby the secondary antibody, the protein A, the enzyme or fragments thereof are deglysosylated.

8. Use of a deglysosylated detection system to identify multiple sugar structures of recombinant antibodies to perform a process according to one of the claims 5 to 7, **characterized in that** the detection system comprises a deglysosylated marker enzyme and a deglycosylated binding structure.

9. Use of a sugar identification kit to perform the process according to one of the claims 5 to 7 comprising at least
- a deglysosylated binding structure selected from the group comprising antibodies, protein A or fragments thereof and
- a deglysosylated enzyme and
- if applicable, information on combining the contents of the kit.

## Revendications

1. Utilisation d'un système de détection déglycosylé pour déterminer en même temps plusieurs structures de sucre d'anticorps recombinants, choisis à partir du groupe comportant IgG, IgM, IgE, IgD, IgA, IgY et/ou IgW,
le système de détection contenant un marqueur enzymatique et une structure liante déglycosylée et les anticorps recombinants étant immobilisés à l'aide de lectines.

2. Utilisation selon la revendication précédente
**caractérisée en ce que**
les structures liantes contiennent au moins des anticorps secondaires, de la protéine A et/ou des fragments de cette dernière.

3. Utilisation selon l'une des revendications précédentes
**caractérisée en ce que**
la détermination des structures de sucres s'effectue à l'aide d'une réaction à substrat de marqueur enzymatique, l'enzyme étant déglycosylé.

4. Utilisation selon l'une des revendications précédentes
**caractérisée en ce que**
l'enzyme déglycosylé est choisi à partir du groupe de phosphatase alcaline, de péroxidase de raifort, de β-galactosidase et/ou d'uréase.

5. Procédé pour déterminer plusieurs structures de sucre d'anticorps recombinants,
**caractérisé en ce que**
le procédé comprend les opérations suivantes :
a) préparation de l'anticorps recombinant à examiner, l'anticorps étant présenté immobilisé, de préférence avec une lectine déglycosylée :
b) incubation avec une structure liante déglycosylée marquée, de préférence un anticorps secondaire, de la protéine A ou un fragment de cette dernière ;
c) détermination de l'interaction entre l'anticorps et la structure liante avec une réaction à substrat de marqueur enzymatique, l'enzyme étant déglycosylé ; et
d) la lectine est présentée immobilisée sur une plaque microtitre.

6. Procédé selon la revendication 5 **caractérisé en ce que**
les anticorps recombinants sont utilisés sans marquage.

7. Procédé selon l'une des revendications 5 ou 6 **caractérisé en ce que** le procédé comprend les opérations suivantes :
a) liaison d'au mois une lectine sur la plaque microtitre ;
b) mise en contact de la lectine avec l'anticorps recombinant à examiner ;
c) incubation avec une structure liante marquée, comprenant au moins un anticorps secondaire, de la protéine A, un marqueur enzymatique ou des fragments de ces derniers ;
d) preuve de la structure liante marquée à l'aide d'une réaction à substrat de marqueur enzymatique, l'anticorps secondaire, la protéine A, l'enzyme ou des fragments de ces derniers étant déglycosylées.

8. Utilisation d'un système de détection déglycosylé pour déterminer plusieurs structures de sucre d'anticorps recombinants pour exécuter un procédé selon l'une des revendications 5 à 7
**caractérisée en ce que**
le système de détection comprend un marqueur enzymatique déglycosylé et une structure liante déglycosylée.

9. Utilisation d'un kit de détermination de sucre pour exécuter le procédé selon l'une des revendications 5 à 7 comprenant au moins
- une structure liante déglycosylée, choisie à partir du groupe comprenant des anticorps secondaire, la protéine A ou des fragments de ces derniers.
- un enzyme déglycosylé et
- le cas échéant une information pour combiner les contenus du kit.
